Europäisches Patentamt

European Patent Office

Office européen des brevets

(11) Publication number: **0 193 984**
**B1**

(12) EUROPEAN PATENT SPECIFICATION

(45) Date of publication of patent specification: **23.01.91**

(51) Int. Cl.⁵: **A 61 K 9/20, A 61 K 9/16**

(21) Application number: **86200256.5**

(22) Date of filing: **19.02.86**

(54) Coprocessed microcrystalline cellulose and calcium carbonate composition and its preparation.

(30) Priority: **08.03.85 US 709748**

(43) Date of publication of application:
**10.09.86 Bulletin 86/37**

(45) Publication of the grant of the patent:
**23.01.91 Bulletin 91/04**

(84) Designated Contracting States:
**BE CH DE FR GB IT LI NL SE**

(56) References cited:
**WO-A-81/02521**

**CHEMICAL ABSTRACTS, vol. 81, no. 16, 21st October 1974, page 324, abstract no. 96411f, Columbus, Ohio, US; J.F. BAVITZ et al.: "Direct compression vehicles. Evaluation of some common diluents", & DRUG COSMET. IND. 1974, 114(4), 44,46,48,50,72**

(73) Proprietor: **FMC Corporation**
**2000 Market Street**
**Philadelphia Pennsylvania 19103 (US)**

(72) Inventor: **Mehra, Dev K.**
**3702 Baring Street**
**Philadelphia Pennsylvania 19104 (US)**
Inventor: **West, Kenneth P.**
**138 Colket Lane**
**Devon Pennsylvania 19333 (US)**
Inventor: **Wiggins, Donald J.**
**18 Canoe Brook Road**
**Princeton Junction New Jersey 08550 (US)**

(74) Representative: **Kooy, Leendert Willem et al**
**OCTROOIBUREAU VRIESENDORP & GAADE**
**P.O. Box 266**
**NL-2501 AW The Hague (NL)**

Courier Press, Leamington Spa, England.

## Description

This invention relates to a particulate composition that contains microcrystalline cellulose and calcium carbonate and which is useful as an excipient in pharmaceutical formulations.

More particularly, microcrystalline cellulose and calcium carbonate are processed together in an aqueous medium and dried to yield a particulate excipient product.

Microcrystalline cellulose is a purified, partially depolymerized cellulose that is prepared by treating alpha cellulose, in the form of a pulp manufactured from fibrous plant material, with mineral acids. It is a white, odorless, tasteless, relatively free flowing powder that is insoluble in water, organic solvents, dilute alkalis and dilute acids. U.S. Patents No. 2,978,446 issued to Battista et al. and No. 3,146,168 issued to Battista are basic patents describing microcrystalline cellulose and its manufacture; the latter patent concerns microcrystalline cellulose for pharmaceutical applications.

Microcrystalline cellulose finds widespread use as a pharmaceutical excipient, an inactive (non-drug) ingredient in pharmaceutical formulations. Its inherent compressibility characteristics account for its popularity as a pharmaceutical excipient, since good binding and disintegration properties are obtained with microcrystalline cellulose when used in direct compression tablet formulations.

Compared to other tablet additives like partially pregelatinized cornstarch, lactose, and dicalcium phosphate, microcrystalline cellulose exhibits superior compressibility and disintegration properties. Unlike these additives, microcrystalline cellulose is relatively costly to manufacture; this limits its use in price-sensitive formulations like vitamins.

A lower cost excipient which has tabletting characteristics similar to those of microcrystalline cellulose would satisfy a need for an economical excipient with good performance that is desired by the vitamin market.

Physical blends of microcrystalline cellulose with starch, lactose or dicalcium phosphate, do not provide the desired performance characteristics. Microcrystalline cellulose coprocessed with either starch or calcium sulfate, as described by Schwartz et al. in Drug & Cosmetic Industry 118, 60 (April 1976) and 114, 44 (April 1974), offers direct compression tabletting performance that falls short of that for microcrystalline cellulose alone.

WO-A-8.102.521 discloses a direct compression vehicle for tabletting of active medicinal and food nutrient materials comprising a blend of powdered cellulose and dicalcium phosphate dihydrate in a ratio of from 85:15 to 15:85, said blend having been compacted in a roller compactor. A portion of the dicalcium phosphate dihydrate may be replaced by e.g. calcium carbonate. Further, a portion of the powdered cellulose may be replaced by e.g. microcrystalline cellulose. However, the compositions obtained by dry blending microcrystalline cellulose and calcium carbonate cannot be compressed to form tablets because such blends will not flow on the platens of a tabletting machine.

The present invention accomplishes the above objective with a composition comprising microcrystalline cellulose and calcium carbonate and prepared in a manner that yields a particulate product with unexpectedly good excipient performance characteristics.

Consequently, the present invention provides a composition of matter useful as a pharmaceutical excipient comprising microcrystalline cellulose and calcium carbonate in a weight ratio of from 75:25 to 35:65, characterized in that the microcrystalline cellulose and calcium carbonate are present as dried particulate agglomerates prepared by forming a well-dispersed aqueous admixture of microcrystalline cellulose and calcium carbonate in particulate form and drying the admixture.

The particulate coprocessed composition is preferably a spray dried material. Particle size of the coprocessed product should be substantially all less than No. 60 Sieve (250 μm) and preferably has an average particle size in the range of from 20 μm to 150 μm.

The particulate coprocessed composition is prepared by forming a well-dispersed aqueous slurry of microcrystalline cellulose and calcium carbonate, both being present in particulate form and in amounts which provide a component ratio in the range of from 75:25 to 35:65 microcrystalline cellulose: calcium carbonate for the coprocessed product; and drying the aqueous slurry by removing water therefrom, to yield a particulate coprocessed product.

The aqueous well-dispersed slurry of the two components is preferably formed by introducing microcrystalline cellulose and calcium carbonate into an aqueous medium, with their addition being in the order mentioned, in amounts that yield a relatively concentrated slurry of at least 10 wt% solids. The aqueous slurry is preferably dried by spray drying to yield the particulate coprocessed product.

The particulate coprocessed product of this invention contains two essential components, microcrystalline cellulose and calcium carbonate. The two components are present in the product in a weight ratio in the range of about 75:25 to 35:65 microcrystalline cellulose: calcium carbonate. The ratio of the two components is preferably in the range of about 70:30 to 40:60 microcrystalline cellulose: calcium carbonate, and most preferably, in the range of about 65:35 to 50:50 microcrystalline cellulose: calcium carbonate.

Other ingredients may also be incorporated into the particulate product during its preparation. These are ordinarily present in relatively small amounts, representing less than 20%, and preferably less than 10%, of the total particulate product weight. Such additives may be incorporated to facilitate the coprocessing procedure, particularly during the drying step, or to provide enhanced properties for the particulate product in its use as a pharmaceutical excipient. Examples of additives in these categories are binders, e.g., water-soluble gums like hydroxypropylmethylcellulose, methylcellulose, polyvinylpyrrolidone, etc.; lubricants, e.g., long chain fatty acid esters or salts thereof like palmitic and stearic acids; and disintegrants like cross-linked carboxymethylcellulose, starch, etc.

The particulate coprocessed product of this invention possesses desirable performance attributes that are not shown with the corresponding dry-blend of microcrystalline cellulose and calcium carbonate. The mechanism that occurs during the coprocessing procedure required in this invention is not fully understood but appears to yield a particulate product in which the two essential components are in intimate association with each other. This intimate association or admixture of microcrystalline cellulose and calcium carbonate cannot be achieved through simple dry blending of these materials, but rather requires that they be coprocessed as an aqueous slurry or mixture.

This intimate association of the two components manifests itself in the appearance of agglomerated particles, containing both microcrystalline cellulose and calcium carbonate, that result after drying of the slurry.

Various characteristics of the particulate coprocessed product, e.g. its particle size, moisture content, bulk density, will be described in detail below, in the context of the process by which this particulate product may be prepared. These physical characteristics are in large measure dependent on the manner in which the microcrystalline cellulose and calcium carbonate are coprocessed. It is for this reason that the drying step in the coprocessing procedure is especially critical, and it is the reason that spray-drying is the preferred method for accomplishing the drying step.

In simple terms, the process for preparing the particulate product of this invention involves forming a well-dispersed aqueous slurry of microcrystalline cellulose and calcium carbonate, in which both materials are present as particulate solids. The relative amounts of the two components are adjusted in the slurry to yield the specific weight ratio desired in the recovered coprocessed product. Since the weight ratio of the two components in the particulate coprocessed product corresponds closely to that in the precursor well-dispersed slurry, this ratio adjustment is relatively straightforward.

The process of this invention next involves drying the aqueous slurry by removing water from it to yield the particulate coprocessed product. As mentioned earlier, spray drying is the preferred drying means but other drying methods, e.g. flash drying or fluidized bed drying, may also be adapted for use in this coprocessing step.

The two components employed in forming the well dispersed aqueous slurry are microcrystalline cellulose and calcium carbonate. The source and nature of these components are not critical. The microcrystalline cellulose is preferably wet cake from a conventional microcrystalline cellulose manufacturing process. The wet cake is material which has not yet been dried, sometimes termed "never dried" or hydrocellulose, to yield a conventional microcrystalline cellulose freeflowing powder product. The microcrystalline cellulose source may also be conventional product, which has already been dried.

Particle size of the microcrystalline cellulose used in the aqueous slurry is ordinarily that which is encountered in conventional microcrystalline cellulose product, or in its precursor wet cake, i.e., never dried product. The particle size is desirably such that substantially all particles are less than No. 60 sieve (250 µm) in size.

Specific size requirements for fine particle sizes, if desired, can be met through screening off unwanted coarse material or through conventional wet or dry attrition procedures. Such attrition may also be accomplished with the microcrystalline cellulose in the aqueous slurry. These size reduction procedures are ordinarily not required with microcrystalline cellulose as is now commercially produced.

The calcium carbonate ($CaCO_3$) employed in this invention is preferably a precipitated material. Precipitated calcium carbonate is ordinarily more pure than ground calcium carbonate and typically has a finer particle size. Ground calcium carbonate may nevertheless be used as a source with satisfactory results.

The particulate calcium carbonate is preferably finer in particle size than the particulate microcrystalline cellulose with which it is coprocessed. Extremely fine particle size calcium carbonate is more readily combined in intimate association with the microcrystalline cellulose during coprocessing of the two components.

Calcium carbonate sizing is preferably such that substantially all particles are less than 30 µm in size and, more preferably, less than 10 µm. Average particle size of the calcium carbonate is desirably less than 5 µm and, more preferably, is less than 2 µm.

Both microcrystalline cellulose and calcium carbonate, it should be recognized, are substantially insoluble in water. Consequently, the particle size of the material present in the well-dispersed aqueous slurry is directly related to the sizing of the two components introduced to the slurry; i.e., there is no appreciable dissolution of either of the two components in the aqueous slurry.

The aqueous slurry of these two components may be prepared in any of several ways. The two solid components may both be introduced into a single aqueous medium, or each may be introduced separately into separate aqueous media which are then combined, or other analogous procedures may be devised.

A preferred procedure involves dispersing the microcrystalline cellulose alone into an aqueous solution, preferably water. Typical solids concentrations for this aqueous mixture are from 5—25 wt% microcrystalline cellulose but 10—20 wt% microcrystalline cellulose is preferred.

Once the microcrystalline cellulose is well-dispersed in the aqueous slurry, the appropriate amount of calcium carbonate is then added, in dry form, with mixing being continued during its addition. The exact amount of calcium carbonate to be added depends on the microcrystalline cellulose content of the slurry and the ratio of the two components desired in the coprocessed product. Water may also be added if a more dilute slurry is desired, but this is usually not required.

The aqueous slurry containing the two components should be well mixed to assure uniform dispersion of the components throughout the aqueous medium. This is necessary to provide for a uniform, consistent component ratio in the particulate product, prepared via drying the aqueous slurry.

The total solids content of the aqueous slurry is preferably at least 10 wt %, based on the total slurry weight, and is more preferably at least 20 wt% solids. The higher solids content levels are desirable since the amount of water that must be removed during the drying step is accordingly reduced.

The upper limit on solids content in the aqueous slurry is typically determined by the operating constraints of the drying apparatus used. With the preferred spray drying procedure, solids contents of 20—30 wt% are representative for aqueous slurries that can be readily processed.

Temperature of the aqueous slurry is not critical. Ambient temperatures, of from about 10—25°C, are preferred. Higher slurry temperatures may be employed, and these may be desirable with certain types of drying equipment.

The drying of the well-dispersed aqueous slurry is preferably accomplished by spray drying of the slurry. Conventional spray drying equipment may be employed, and operating procedures that are familiar to those experienced in the spray drying art are applicable to the spray drying step of this process. Drier (drier gas) outlet temperature is ordinarily used to control the residual moisture level obtained in the coprocessed particulate product.

Moisture levels of about 1—8 wt% $H_2O$ are desired in the particulate, dried product and moisture levels of about 3—7 wt% are most preferred. These levels are based on the finding that the coprocessed product, if completely dried (no residual moisture), will usually absorb moisture from the atmosphere, in amounts within these specified ranges.

In a spray drying procedure, drier outlet temperatures are ordinarily in the range of about 40—100°C. Corresponding drier inlet temperatures are higher, ordinarily in the range of about 90—300°C.

The coprocessed product recovered from the drying operation is a free-flowing particulate solid, that is typically a granular white powder in appearance. particle size of the product is a function of the particle sizing of the microcrystalline cellulose and calcium carbonate in the aqueous slurry and, more importantly, of the drying conditions employed for removing water from the slurry.

The particulate coprocessed product should have a particle size that is substantially all less than No. 60 sieve (250 µm). Average particle size of the particulate material is preferably in the range of from about 20 µm to 150 µm, and more preferably is in the range of from about 30 µm to 100 µm.

Bulk density (loose) of the coprocessed product, with a preferred component ratio of 60:40 microcrystalline cellulose: calcium carbonate, typically is in the range of about 0.35—0.45 g/cm³; microcrystalline cellulose ordinarily exhibits a loose bulk density of around 0.28—0.30 g/cm³.

An aqueous slurry of the coprocessed product with the preferred component ratio of 60:40 exhibits a moderately alkaline pH around 9.5—10, whereas microcrystalline cellulose in an aqueous slurry has a pH of around 6.5—7.

The particulate coprocessed product of this invention, besides being economical, has several desirable properties that make it particularly well-suited for use as an excipient for vitamins, in direct compression tabletting applications.

The coprocessed microcrystalline cellulose/calcium carbonate product exhibits low lubricant sensitivity; its compression profile (tablet hardness vs. tablet compression force) remains relatively unchanged when various lubricants are employed with the excipient. This lubricant insensitivity extends both to lubricant level (amount) and lubricant type (magnesium stearate, stearic acid, etc.). The compression force required to produce a specific tablet hardness does not change when lubricants are employed with the coprocessed excipient of this invention.

By contrast, the compression profile for pure microcrystalline cellulose is adversely affected by the introduction of lubricants, with higher compression forces being required to produce the same tablet hardness as for the equivalent lubricant-free tablets.

Flow characteristics of the particulate coprocessed material are good, and its use in conventional direct compression tabletting equipment does not present operating difficulties due to the material's particle flow behavior.

The compressibility of this coprocessed product compares favorably with that of commercially available microcrystalline celluloses. Compressibility is typically measured as the profile, or shape, of the plot of tablet hardness vs. tablet compression force. Compressibility of a excipient is desirably high, since low levels of excipient may then be used in tabletting an active ingredient (with concurrent larger amounts of active ingredient being present) without compromising tablet performance characteristics.

The compressibility profile of a 60:40 microcrystalline cellulose: calcium carbonate coprocessed product is very similar to that of microcrystalline cellulose available from Ming Tai and designated 101 grade. Both of these are less compressible than Avicel® PH-101 microcrystalline cellulose, a commercial product manufactured by FMC Corporation.

The Example describes the preparation of a particulate coprocessed microcrystalline cellulose and calcium carbonate product having the two components present in a weight ratio of about 60:40. The particulate coprocessed composition is prepared by spray drying, a preferred drying procedure.

In the preparation of this product, a well-dispersed aqueous slurry of microcrystalline cellulose and calcium carbonate is first formed. Microcrystalline cellulose, material obtained from a conventional microcrystalline cellulose manufacturing process as moist product filter cake that has not been dried, is introduced into water at about 20°C in a sufficient amount to yield an aqueous slurry containing about 17—18 wt% solids.

The microcrystalline cellulose that is employed to make the slurry is particulate in form, but exact sizing is difficult to measure due to its being a moist filter cake.

Calcium carbonate, a precipitated fine powder having an average particle size of about 1 μm (USP Albaglos precipitated calcium carbonate from Pfizer, Inc.), is introduced into the microcrystalline cellulose slurry in an amount corresponding to two-thirds of the amount (weight) of the microcrystalline cellulose.

The aqueous slurry, containing about 25 wt % solids, is thoroughly mixed so as to assure homogeneous dispersion of the two solid components throughout the slurry.

The well-dispersed slurry is then fed continuously to a Koch 40 ft. diameter spray drier, in which the drier inlet and outlet temperatures are maintained at about 180°C and 70°C, respectively.

The particulate spray-dried coprocessed material recovered from spray drying contains a 59—41 weight ratio of microcrystalline cellulose: calcium carbonate. The free-flowing white powder has a loose bulk density of about 0.39—0.40 g/cm³, is substantially all smaller than No. 60 sieve (250 μm), and has an average particle size of about 61 μm. The spray drying temperature conditions and slurry feed rate yield a particulate product that contains about 3 wt % $H_2O$.

Particulate spray-dried coprocessed product prepared in the manner just described may be employed as an excipient in vitamin or other pharmaceutical formulations intended for direct compression tabletting. The procedure for such use in direct compression tabletting is conventional: blend the excipient in the desired amount with the other dry ingredients to be tabletted; add lubricants, if desired and continue blending a short period longer, e.g., five minutes; and compress the dry blend at the desired hardness setting in a tabletting machine.

## Claims

1. A composition of matter useful as a pharmaceutical excipient comprising microcrystalline cellulose and calcium carbonate in a weight ratio of from 75:25 to 35:65, characterized in that the microcrystalline cellulose and calcium carbonate are present as dried particulate agglomerates prepared by forming a well-dispersed aqueous admixture of microcrystalline cellulose and calcium carbonate in particulate form and drying the admixture.

2. The composition of claim 1, characterized in that the particulate agglomerates are prepared by spray drying the aqueous admixture of microcrystalline cellulose and calcium carbonate.

3. The composition of claim 1, characterized in that the weight ratio of the two components is in the range of from 70:30 to 40:60 microcrystalline cellulose: calcium carbonate.

4. The composition of claim 1, characterized in that the weight ratio of the two components is in the range of from 65:35 to 50:50 microcrystalline cellulose: calcium carbonate.

5. The composition of claim 1, characterized in that the particle size of the particulate agglomerates of microcrystalline cellulose and calcium carbonate is substantially all less than No. 60 Sieve (250 μm).

6. The composition of claim 1, characterized in that the particulate agglomerates of microcrystalline cellulose and calcium carbonate have an average particle size in the range of from 20 μm to 150 μm.

7. The composition of claim 1, characterized in that the particulate agglomerates of microcrystalline cellulose and calcium carbonate have a moisture content of from 1—8 wt% $H_2O$.

8. A process for preparing a particulate material useful as a pharmaceutical excipient, characterized by dispersing microcrystalline cellulose and calcium carbonate, both in particulate form, into an aqueous medium to form a well-dispersed aqueous slurry having a total solids content of at least 10 wt % based on the total slurry weight and containing relative amounts of microcrystalline cellulose and calcium carbonate that provide a component ratio within the range specified below for the particulate coprocessed product, and drying the aqueous slurry by removing water therefrom to yield a particulate coprocessed product, in which the weight ratio of microcrystalline cellulose to calcium carbonate is in the range of from 75:25 to 35:65.

9. The process of claim 8, characterized in that the aqueous slurry is dried by spray drying.

10. The process of claim 8, characterized in that the weight ratio of microcrystalline cellulose to calcium carbonate is in the range of from 65:35 to 50:50.

11. The process of claim 8, characterized in that the drying conditions are adjusted to yield a dried particulate coprocessed product having a residual moisture content of from 1—8 wt% $H_2O$.

12. The process of claim 8, characterized in that the drying conditions are adjusted to yield a dried particulate coprocessed product having particle size that is substantially all less than No. 60 Sieve (250 μm).

13. The process of claim 12, characterized in that the dried particulate coprocessed product has an average particle size in the range of from 20 μm to 150 μm.

14. The process of claim 8, characterized in that the particulate microcrystalline cellulose is never-dried material from a microcrystalline cellulose manufacturing process.

15. The process of claim 8, characterized in that the particulate calcium carbonate in the aqueous slurry is substantially finer than the particulate microcrystalline cellulose therein.

16. The process of claim 8, characterized in that the particulate microcrystalline cellulose in the aqueous slurry has a particle size that is substantially all less than No. 20 Sieve (250 μm).

17. The process of claim 8, characterized in that the particulate calcium carbonate is substantially all smaller than 30 μm.

18. The process of claim 8, characterized in that the particulate calcium carbonate is substantially all smaller than 10 μm.

19. The process of claim 8, characterized in that the average particle size of the particulate calcium carbonate is less than 5 μm.

20. The process of claim 8, characterized in that the well-dispersed aqueous slurry to be dried has a solids content of at least 20 wt % solids, based on the total slurry weight.

21. The process of claim 9, characterized in that the spray drying operation is carried out with a drier outlet temperature in the range of from 40—100°C.

22. The process of claim 9, characterized in that the spray drying operation is carried out with a drier inlet temperature in the range of from 90—300°C.

**Patentansprüche**

1. Eine Zusammensetzung die als einen pharmazeutischen Excipient brauchbar ist und eine mikrokristalline Zellulose und Calciumcarbonat in einem Gewichtsverhältnis von 75:25 bis 35:65 enthält, dadurch gekennzeichnet, dass die mikrokristalline Zellulose und Calciumcarbonat als getrockene aus Teilchen bestehenden Agglomerate anwesend sind, die hergestellt sind durch eine gut dispergierte wässerige Mischung von mikrokristalliner Zellulose und Calciumcarbonat in einer aus Teilchen bestehender Form zu bilden und die Mischung zu trocknen.

2. Die Zusammensetzung nach Anspruch 1, dadurch gekennzeichnet, dass die aus Teilchen bestehenden Agglomerate hergestellt sind durch die wässerige Mischung von mikrokristalliner Zellulose und Calciumcarbonat zu sprühtrocknen.

3. Die Zusammensetzung nach Anspruch 1, dadurch gekennzeichnet, dass das Gewichtsverhältnis der beiden Komponenten in dem Bereich von 70:30 bis 40:60 mikrokristalline Zellulose: Calciumcarbonat ist.

4. Die Zusammensetzung nach Anspruch 1, dadurch gekennzeichnet, dass das Gewichtsverhältnis der beiden Komponenten in dem Bereich von 65:35 bis 50:50 mikrokristalline Zellulose: Calciumcarbonat ist.

5. Die Zusammensetzung nach Anspruch 1, dadurch gekennzeichnet, dass die Teilchengrösse der aus Teilchen bestehenden Agglomerate von mikrokristalliner Zellulose und Calciumcarbonat im wesentlichen weniger als Nr. 60 Sieb (250 μm) ist.

6. Die Zusammensetzung nach Anspruch 1, dadurch gekennzeichnet, dass die aus Teilchen bestehenden Agglomerate von mikrokristalliner Zellulose und Calciumcarbonat eine durchschnittliche Teilchengrösse in dem Bereich von 20 μm bis 150 μm haben.

7. Die Zusammensetzung nach Anspruch 1, dadurch gekennzeichnet, dass die aus Teilchen bestehenden Agglomerate von mikrokristalliner Zellulose und Calciumcarbonat einen Feuchtigkeitsgehalt von 1—8 Gewichtsprozent $H_2O$ haben.

8. Ein Verfahren zur Herstellung ein aus Teilchen bestehendes Material das brauchbar ist als einen pharmazeutischen Excipient, dadurch gekennzeichnet, dass man mikrokristalline Zellulose und Calciumcarbonat, beide in einer aus Teilchen bestehender Form, in einem wässerigen Medium dispergiert um eine gut dispergierte wässerige Suspension zu bilden, die einen Feststoffgehalt von mindestens 10 Gewichtsprozent hat, basiert auf das totale Suspensionsgewicht, und relative Mengen von mikrokristalliner Zellulose und Calciumcarbonat enthält, die ein Komponentsverhältnis innerhalb dem Bereich wie hiernach angegeben verschaffen für das aus Teilchen bestehende, gleichzeitig hergestellte Produkt, und die wässerige Suspension trocknet durch Wasser davon zu entfernen um ein aus Teilchen bestehendes, gleichzeitig hergestelltes Produkt zu gewinnen, in dem das Gewichtsverhältnis von mikrokristalliner Zellulose zu Calciumcarbonat in dem Bereich von 75:25 bis 35:65 ist.

9. Das Verfahren nach Anspruch 8, dadurch gekennzeichnet, dass die wässerige Suspension durch sprühtrocknen getrocknet wird.

10. Das Verfahren nach Anspruch 8, dadurch gekennzeichnet, dass das Gewichtsverhältnis von mikrokristalliner Zellulose zu Calciumcarbonat in dem Bereich von 65:35 bis 50:50 ist.

11. Das Verfahren nach Anspruch 8, dadurch gekennzeichnet, dass die Umstände beim Trocknen angepasst werden um ein getrocknetes aus Teilchen bestehendes, gleichzeitig hergestelltes Produkt zu gewinnen, das eine restliche Feuchtigkeitsgehalt von 1—8 Gewichtsprozent $H_2O$ hat.

12. Das Verfahren nach Anspruch 8, dadurch gekennzeichnet, dass die Umstände beim Trocknen angepasst werden um ein getrocknetes aus Teilchen bestehendes, gleichzeitig hergestelltes Produkt zu gewinnen, das eine Teilchengrösse hat die im wesentlichen geringer ist als Nr. 60 Sieb (250 μm).

13. Das Verfahren nach Anspruch 12, dadurch gekennzeichnet, dass das getrocknete aus Teilchen bestehende, gleichzeitig hergestellte Produkt eine durchschnittliche Teilchengrösse in dem Bereich von 20 μm bis 150 μm hat.

14. Das Verfahren nach Anspruch 8, dadurch gekennzeichnet, dass die aus Teilchen bestehende mikrokristalline Zellulose ein nie getrocknetes Material von einem Verfahren zur Herstellung mikrokristalliner Zellulose ist.

15. Das Verfahren nach Anspruch 8, dadurch gekennzeichnet, dass das aus Teilchen bestehende Calciumcarbonat in der wässerigen Suspension wesentlich feiner ist als die aus Teilchen bestehende mikrokristalline Zellulose darin.

16. Das Verfahren nach Anspruch 8, dadurch gekennzeichnet, dass die aus Teilchen bestehende mikrokristalline Zellulose in der wässerigen Suspension eine Teilchengrösse hat, die im wesentlichen geringer ist als Nr. 20 Sieb (250 μm).

17. Das Verfahren nach Anspruch 8, dadurch gekennzeichnet, dass das aus Teilchen bestehende Calciumcarbonat im wesentlichen kleiner ist als 30 μm.

18. Das Verfahren nach Anspruch 8, dadurch gekennzeichnet, dass das aus Teilchen bestehende Calciumcarbonat im wesentlichen kleiner ist als 10 μm.

19. Das Verfahren nach Anspruch 8, dadurch gekennzeichnet, dass die durchschnittliche Teilchengrösse des aus Teilchen bestehenden Calciumcarbonats geringer ist als 5 μm.

20. Das Verfahren nach Anspruch 8, dadurch gekennzeichnet, dass die gut dispergierte wässerige Suspension, die getrocknet werden muss, einen Feststoffgehalt von mindestens 20 Gewichtsprozent hat, basiert auf das totale Suspensionsgewicht.

21. Das Verfahren nach Anspruch 9, dadurch gekennzeichnet, dass das Sprühtrocknen bei einer Temperatur des Trocknerauslasses in dem Bereich von 40—100°C ausgeführt wird.

22. Das Verfahren nach Anspruch 9, dadurch gekennzeichnet, dass das Sprühtrocknen bei einer Temperatur des Trocknereinlasses in dem Bereich von 90—300°C ausgeführt wird.

**Revendications**

1. Composition d'une matière utile comme excipient pharmaceutique comprenant de la cellulose microcristalline et du carbonate de calcium selon un rapport pondéral allant de 75:25 à 35:65, caractérisée en ce que la cellulose microcristalline et le carbonate de calcium sont présents sous forme d'agglomérats de particules sèchées, préparés en formant un mélange aqueux bien dispersé de cellulose microcristalline et de carbonate de calcium sous forme de particules et en séchant le mélange.

2. Composition selon la revendication 1, caractérisée en ce que les agglomérats de particules sont préparés par séchage par pulvérisation du mélange aqueux de cellulose microcristalline et de carbonate de calcium.

3. Composition selon la revendication 1, caractérisée en ce que le rapport pondéral des deux constituants cellulose microcristalline: carbonate de calcium est compris dans l'intervalle allant de 70:30 à 40:60.

4. Composition selon la revendication 1, caractérisée en ce que le rapport pondéral des deux constituants cellulose microcristalline: carbonate de calcium est compris dans l'intervalle allant de 65:35 à 50:50.

5. Composition selon la revendication 1, caractérisée en ce que les tailles des particules des agglomérats particulaires de cellulose microcristalline et de carbonate de calcium sont sensiblement toutes inférieures au tamis No. 60 (250 microns).

6. Composition selon la revendication 1, caractérisée en ce que les agglomérats particulaires de cellulose microcristalline et de carbonate de calcium possèdent une taille de particule moyenne comprise dans l'intervalle allant de 20 micromètres à 150 micromètres.

7. Composition selon la revendication 1, caractérisée en ce que les agglomérats particulaires de, cellulose microcristalline et de carbonate de calcium ont une teneur en humidité d'environ 1—8% en poids d'eau.

8. Procédé pour la préparation d'une substance particulaire utile comme excipient pharmaceutique, caractérisé en ce qu'il consiste à disperser de la cellulose microcristalline et du carbonate de calcium, tous deux sous forme de particules, dans un milieu aqueux pour former une suspension aqueuse bien dispersée possédant une teneur en matières solides totale d'au moins 10% en poids basée sur le poids total de la suspension et contenant des quantités relatives de cellulose microcristalline et de carbonate de calcium qui procurent un rapport de constituants situé dans l'intervalle spécifié ci-essous pour le produit co-préparé en particules, et à sécher la suspension aqueuse en éliminant l'eau de celle-ci pour conduire à un produit co-préparé en particules dans lequel le rapport pondéral de la cellulose microcristalline au carbonate de calcium est compris dans l'intervalle allant de 75:25 à 35:65.

9. Procédé selon la revendication 8, caractérisé en ce que la suspension aqueuse est séchée par un séchage par pulvérisation.

10. Procédé selon la revendication 8, caractérisé en ce que le rapport pondéral de la cellulose microcristalline et du carbonate de calcium est compris dans l'intervalle allant de 65:35 à 50:50.

11. Procédé selon la revendication 8, caractérisé en ce que les conditions de séchage sont ajustées pour conduire à un produit co-préparé en particules séché possédant une teneur en humidité résiduelle d'environ 1—8% en poids d'$H_2O$.

12. Procédé selon la revendication 8, caractérisé en ce que les conditions de séchage sont ajustées pour obtenir un produit co-préparé en particules séché possédant des tailles de particules qui sont sensiblement toutes inférieures au tamis No. 60 (250 micromètres).

13. Procédé selon la revendication 12, caractérisé en ce que le produit co-préparé en particules séché possède une taille de particule moyenne comprise dans l'intervalle allant de 20 micromètres à 150 micromètres.

14. Procédé selon la revendication 8, caractérisé en ce que la cellulose microcristalline particulaire est une matière "non séchée" issue d'un procédé de fabrication de cellulose microcristalline.

15. Procédé selon la revendication 8, caractérisé en ce que le carbonate de calcium particulaire dans la suspension aqueuse est sensiblement plus fin que la cellulose microcristalline particulaire dans celle-ci.

16. Procédé selon la revendication 8, caractérisé en ce que la cellulose microcristalline en particules dans la suspension aqueuse possède des tailles de particules qui sont sensiblement toutes inférieures au tamis No. 20 (250 micromètres).

17. Procédé selon la revendication 8, caractérisé en ce que les particules de carbonate de calcium sont sensiblement toutes plus petites que 30 micromètres.

18. Procédé selon la revendication 8, caractérisé en ce que les particules de carbonate de calcium sont sensiblement toutes plus petites que 10 micromètres.

19. Procédé selon la revendication 8, caractérisé en ce que la taille de particule moyenne du carbonate de calcium particulaire est inférieure à 5 micromètres.

20. Procédé selon la revendication 8, caractérisé en ce que la suspension aqueuse bien dispersée à sécher possède une teneur en matières solides d'au moins 20% en poids de solides, basée sur le poids total de la suspension.

21. Procédé selon la revendication 9, caractérisé en ce que l'opération de séchage par pulvérisation est effectuée avec une température de sortie de sécheur comprise dans l'intervalle 40—100°C.

22. Procédé selon la revendication 9, caractérisé en ce que l'opération de séchage par pulvérisation est effectuée avec une température d'entrée de sécheur comprise dans l'intervalle 90—300°C.